# EUROPÄISCHE PATENTSCHRIFT

(11) **EP 2 063 799 B1**
(45) Veröffentlichungstag und Bekanntmachung des Hinweises auf die Patenterteilung: **01.09.2010**
(21) Anmeldenummer: 07802228.2
(22) Anmeldetag: 08.09.2007
(51) Int. Cl.: A61B 17/82

(54) **STERNUMVERSCHLUSS**
STERNUM CLOSURE DEVICE
DISPOSITIF DE FERMETURE STERNALE

(30) Priorität: 22.09.2006 DE 102006046424
(43) Veröffentlichungstag der Anmeldung: 03.06.2009
(73) Patentinhaber: Aesculap AG, 78532 Tuttlingen (DE)
(72) Erfinder: MORALES, Pedro, 78532 Tuttlingen (DE); WEISSHAUPT, Dieter, 78194 Immendingen (DE); LUTZE, Theodor, 78582 Balgheim (DE); DWORSCHAK, Manfred, 78589 Dürbheim (DE)
(74) Vertreter: Hoeger, Stellrecht & Partner Patentanwälte
(86) Internationale Anmeldenummer: PCT/EP2007/007848
(87) Internationale Veröffentlichungsnummer: WO 2008/034537

(56) Entgegenhaltungen:
- DE-B4- 10 326 690

## Beschreibung

Die Erfindung betrifft einen Sternumverschluß zur Festlegung von zwei miteinander zu verbindenden Sternumteilen mit einem inneren Anlageelement zur Anlage an der Innenfläche des Sternums, mindestens einem daran festgelegten, quer von diesem abstehenden Spannelement und mit mindestens einem äußeren Anlageelement zur Anlage an der Außenseite des Sternums, welches mittels des durch den Zwischenraum zwischen den Sternumteilen hindurchgeführten Spannelements gegen das innere Anlageelement spannbar ist.

Ein derartiger Sternumverschluß ist beispielsweise in der DE 103 26 690 B4 beschrieben. Er dient dazu, die beiden Teile des Sternums nach einer Durchtrennung desselben in der einander angenäherten Position zu fixieren, so daß die beiden Sternumteile wieder knöchern miteinander verbunden werden können. Ein derartiger Sternumverschluß ist sehr einfach aufgebaut, leicht anzulegen und fixiert die Sternumteile sicher, so daß der Heilungsprozeß gefördert wird.

Im Falle einer erneuten Operation, bei der das Sternum erneut längs einer Trennlinie durchtrennt werden muß, können sich jedoch Schwierigkeiten durch die bekannten Sternumverschlüsse ergeben, da diese eine Trennung, beispielsweise durch Sägen, behindern können und selbst schwer zu entfernen sind, da sie von dem neu gebildeten Knochenmaterial umwachsen sein können.

Es ist Aufgabe der Erfindung, einen gattungsgemäßen Sternumverschluß so auszubilden, daß im Falle der erneuten Durchtrennung des Sternums die Teile des Sternumverschlusses die Durchtrennung möglichst wenig behindern. Diese Aufgabe wird bei einem Sternumverschluß der eingangs beschriebenen Art erfindungsgemäß dadurch gelöst, daß das innere Anlageelement aus zwei voneinander getrennten Teilen besteht, von denen jedes zur Anlage an einem der beiden Sternumteile ausgebildet ist, und daß Verbindungsmittel zur lösbaren Verbindung der beiden nebeneinander angeordneten Teile vorgesehen sind.

Die äußeren Anlageelemente können bei einer Reoperation relativ leicht entfernt werden, es genügt dazu, die Spannelemente abzuheben. Wesentlich schwieriger ist dies bei den inneren Anlageelementen, da diese nicht zugänglich sind. Durch den Aufbau des inneren Anlageelementen aus zwei voneinander getrennten Teilen, von denen jedes einem Sternumteil zugeordnet ist, und durch die lösbare Verbindung dieser beiden Teile des inneren Anlageelementes lassen sich diese beiden Teile nach Lösung der Verbindung voneinander trennen und können somit der Trennung der Sternumteile folgen, wenn diese aufgespreizt werden. Dadurch behindern die inneren Anlageelemente dieses Aufspreizen der beiden Sternumteile nicht.

Jedes Teil des inneren Anlageelementes kann bei der Anlage an der Innenfläche der Sternumteile in diese eindringende Fixiervorsprünge tragen, so daß die beiden Teile des inneren Anlageelementes an den Sternumteilen festgelegt werden können, bevor diese nach einer Operation durch eine Spannvorrichtung gegeneinander gespannt werden.

Günstig ist es, wenn die Teile des inneren Anlageelementes eine im wesentlichen ebene Anlagefläche zur Anlage an der Innenfläche der Sternumteile aufweisen.

Insbesondere können die Spannelemente stiftförmig ausgebildet sein.

Bei einer ersten bevorzugten Ausführungsform ist vorgesehen, daß an jedem der beiden Teile des inneren Anlageelementes Spannelemente angeordnet sind und daß die äußeren Anlageelemente jeweils mit Spannelementen beider Teile des inneren Anlageelementes in Verbindung stehen, so daß die beiden Teile des inneren Anlageelements durch das äußere Anlageelement nebeneinanderliegend zusammengehalten werden.

Die äußeren Anlageelemente bilden somit eine Brücke zwischen den beiden Teilen des innenliegenden Anlageelementes, diese Brücke wird durch die Spannelemente mit den beiden Teilen des inneren Anlageelementes verbunden, so daß einmal durch diese Verbindung und zum anderen durch das äussere Anlageelement die beiden Sternumteile in der zusammengespannten Stellung fixiert werden.

Bei einer erneuten Operation können die äußeren Anlageelemente entfernt werden, und dann sind die beiden Teile des inneren Anlageelementes nicht mehr miteinander verbunden, die Sternumteile können nach Durchtrennung mit einer Knochensäge ohne weiteres aufgespreizt werden.

Es kann vorgesehen sein, daß die Spannelemente in einem Bereich der Teile des inneren Anlageelementes mit diesen verbunden sind, der in Richtung auf das jeweils andere Teil vorspringt, und daß diese vorspringenden Bereiche der Teile des inneren Anlageelementes in Längsrichtung des Zwischenraums zwischen den Sternumteilen gegeneinander versetzt sind. Auf diese Weise ist es möglich, daß die Spannelemente mehr oder weniger linienförmig längs des Zwischenraums zwischen den beiden Sternumteilen angeordnet werden, wobei die die Spannelemente tragenden, jeweils vorspringenden Bereiche der beiden Teile des inneren Anlageelementes abwechselnd diesen Zwischenraum überbrücken.

Die vorspringenden Bereiche können beispielsweise dreieckförmig ausgebildet und im Bereich der Spitze des Dreiecks mit dem Spannelement verbunden sein.

Besonders vorteilhaft ist es, wenn an jedem der beiden Teile des inneren Anlageelementes in Längsrichtung des Zwischenraumes zwischen den Sternumteilen aufeinanderfolgend mehrere Spannelemente angeordnet sind. Die Teile des inneren Anlageelementes können sich über die gesamte Höhe des Sternums erstrecken und tragen dann eine größere Anzahl von aufeinanderfolgenden Verbindungselementen, beispielsweise vier oder fünf Verbindungselemente.

Es ist dabei günstig, wenn mehrere äußere Anlageelemente vorgesehen sind, von denen jedes mit je einem Spannelement des einen und des anderen Teils des inneren Anlageelementes verbunden ist. Während also das innere Anlageelement sich dann über eine größere Länge des Zwischenraums zwischen den Sternumteilen erstreckt, ist dies bei den äußeren Anlageelementen nicht der Fall, diese verbinden nur jeweils je ein Spannelement des einen Teils und des anderen Teils des inneren Anlageelementes.

Bei einer weiteren bevorzugten Ausführungsform ist vorgesehen, daß die beiden Teile des inneren Anlageelementes flächig aneinanderliegende Bereiche aufweisen und daß die Verbindungsmittel die beiden Teile in diesen Bereichen miteinander lösbar verbinden. Man erhält dadurch eine hohe Stabilität des inneren Anlageelementes, und nach Lösung der Verbindungsmittel können beide Teile des inneren Anlageelementes ohne weiteres der Spreizbewegung der Sternumteile folgend voneinander getrennt werden.

Beispielsweise können die beiden Teile durch Gewindeschrauben miteinander verschraubt sein.

Besonders vorteilhaft ist es, wenn die Gewindeschrauben durch die Spannelemente gebildet werden. Bei einer Reoperation können nach Abnahme der äusseren Anlageelemente die Spannelemente aus dem inneren Anlageelement herausgeschraubt werden und lösen dadurch die Verbindung zwischen den beiden Teilen des inneren Anlageelements.

Es ist günstig, wenn die beiden Teile des inneren Anlageelementes auf den dem jeweils anderen Teil zugewandten Seiten zinkenförmige Vorsprünge tragen und wenn diese zinkenförmigen Vorsprünge in Längsrichtung des Zwischenraumes zwischen den Sternumteilen gegeneinander versetzt derart ineinander greifen, daß die zinkenförmigen Vorsprünge des einen Teils jeweils das andere Teil untergreifen. Man erhält dadurch eine hohe Stabilität des inneren Anlageelementes, wenn die beiden Teile durch die Verbindungsmittel gegeneinander gespannt werden.

Bei einer anderen Ausführungsform kann vorgesehen sein, daß eines der beiden Teile des inneren Anlageelementes auf seiner dem anderen Teil zugewandten Seite zinkenförmige Vorsprünge trägt, die in Durchbrechungen des anderen Teils eintauchen und das andere Teil untergreifen.

Vorzugsweise ist das innere Anlageelement mit zwei Spannelementen verbunden, die in Längsrichtung des Zwischenraums zwischen den Sternumteilen versetzt sind. Dadurch ergibt sich eine Positionierhilfe beim Einsetzen der inneren Anlageelemente, und bei Ausführungen, bei denen die Spannelemente die beiden Teile des inneren Anlageelementes lösbar miteinander verbinden, wird sichergestellt, daß die beiden Teile des inneren Anlageelementes unverdrehbar miteinander verbunden werden.

Es kann vorgesehen sein, daß die Anlageelemente mit einem Material beschichtet sind, das die Verbindung mit Knochenmaterial verhindert, beispielsweise mit Polytetrafluorethylen. Dadurch wird das Ablösen der Anlageelemente von umgebendem Knochenmaterial erleichtert, falls eine erneute Durchtrennung des Sternums notwendig ist.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht einer ersten bevorzugten Ausfüh- rungsform eines Sternumverschlusses mit einem aus zwei ge- trennten Teilen bestehenden inneren Anlageelement im angeleg- ten Zustand vor dem Kürzen der stiftförmigen Spannelemente;
- Figur 2:: eine Ansicht ähnlich Figur 1, wobei zwei äußere Anlageelemente und das Knochenmaterial zur Erhöhung der Deutlichkeit wegge- lassen sind;
- Figur 3:: eine perspektivische Ansicht des Sternumverschlusses der Figur 1 vor dem Zusammenfügen der einzelnen Teile;
- Figur 4:: ein weiteres bevorzugtes Ausführungsbeispiel eines Sternum- verschlusses mit teilbarem inneren Anlageelement;
- Figur 5:: eine Seitenansicht des Sternumverschlusses der Figur 4 und
- Figur 6:: eine Ansicht des Sternumverschlusses der Figur 4 von unten.

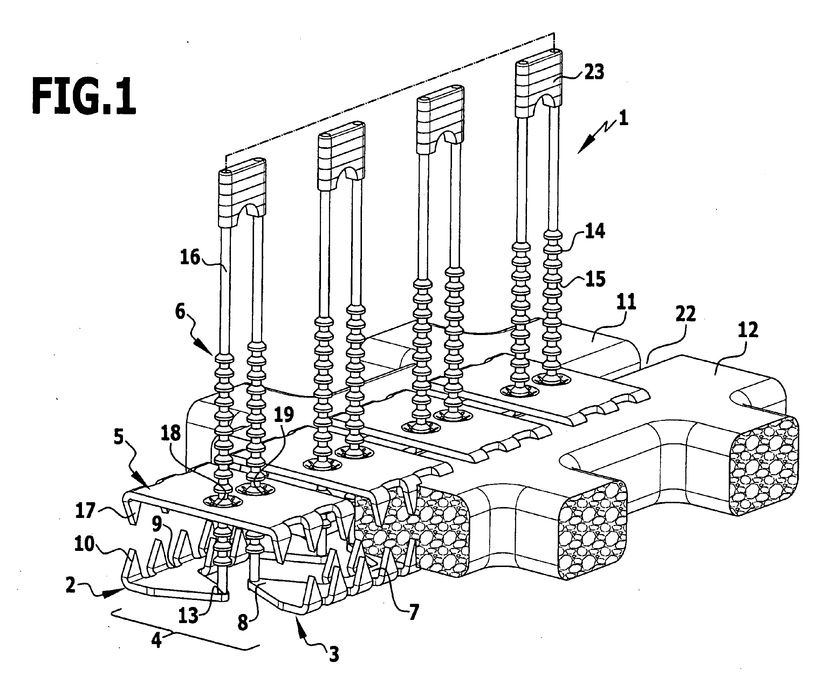

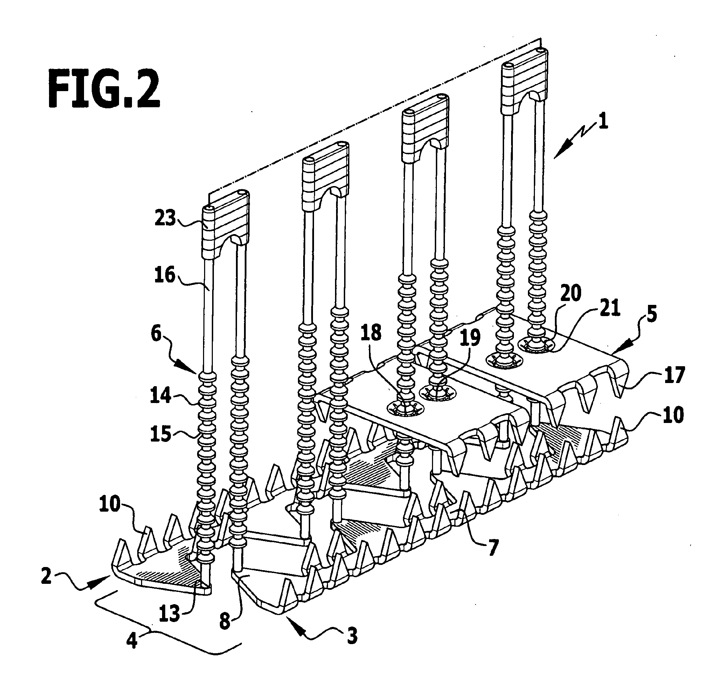

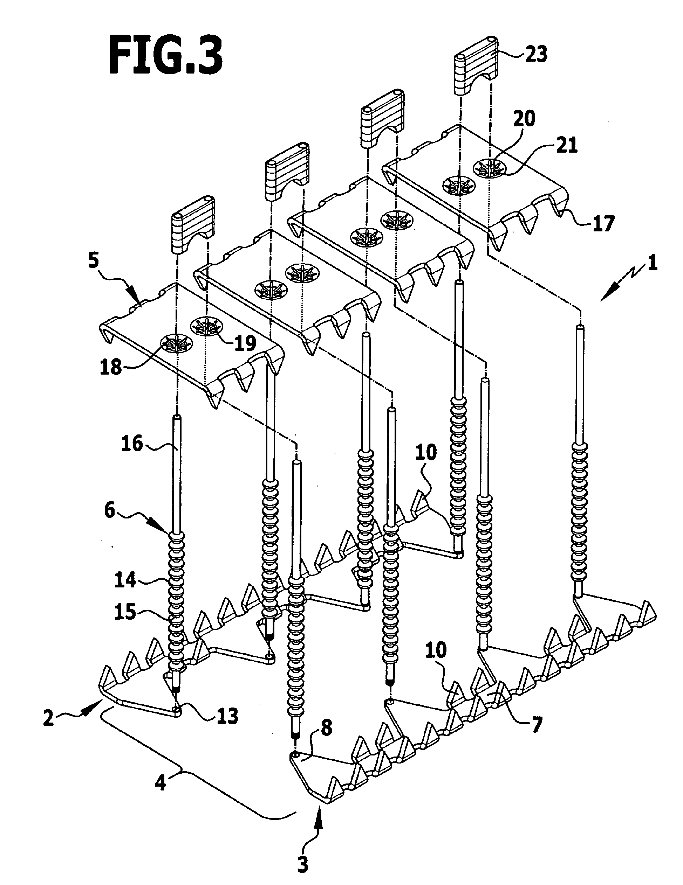

Der in den Figuren 1 bis 3 dargestellte Sternumverschluß 1 umfaßt ein aus zwei Teilen 2, 3 bestehendes inneres Anlageelement 4, eine Anzahl von äußeren Anlageelementen 5 sowie an den Teilen 2, 3 des inneren Anlageelementes 4 festgelegte Raststift 6. Die beiden Teile 2, 3 des inneren Anlageelementes 4 sind gleich aufgebaut, daher wird nachstehend nur eines der beiden näher beschrieben. Das Teil 2 weist einen länglichen, plattenförmigen und ebenen Steg 7 auf, von dem nach einer Seite dreieckförmige Verbindungsbereiche 8 abstehen, die in Längsrichtung des Steges 7 im Abstand zueinander angeordnet sind. Zwischen den Verbindungsbereichen 8 einerseits und auf der gegenüberliegenden, von den Verbindungsbereichen 8 abgewandten Längsseite 9 des Steges 7 andererseits stehen senkrecht zur Fläche des Steges 7 nach oben umgebogene spitze Vorsprünge 10 ab, die der Fixierung der Teile 2, 3 an der Innenfläche eines Sternumteiles 11 bzw. 12 dienen.

Im Bereich der Spitzen der dreieckförmigen Verbindungsbereiche 8 sind diese mit den Raststiften 6 verbunden, die Raststifte 6 stehen senkrecht von der Fläche der Stege 7 nach oben ab und können beispielsweise in Gewindebohrungen 13 der Verbindungsbereiche 8 eingeschraubt oder mit diesen vernietet oder in anderer Weise verbunden sein. Die Raststifte 6 weisen in ihrem unteren Teil einen mit Umfangsrippen 14 versehenen Rastabschnitt 15 und einen daran anschließenden, glatten Verlängerungsabschnitt 16 auf.

Die äußeren Anlageelemente 5 sind rechteckig und plattenförmig ausgebildet, an zwei gegenüberliegenden Kanten weisen sie senkrecht abgebogene, spitze Vorsprünge 17 auf, die ebenfalls zum Eingriff in den Sternumteile 11, 12 vorgesehen sind. In dem ebenen Teil der äußeren Anlageelemente 5 sind zwei gleich ausgebildete Öffnungen 18, 19 angeordnet, deren Ränder radial nach außen verlaufende Einschnitte 20 aufweisen, so daß die Randbereiche in getrennt voneinander verbiegbare Lappen 21 unterteilt sind.

Die Anlageelemente und die Raststifte werden aus Metall gefertigt, dabei werden körperverträgliche Metalle verwendet, beispielsweise Titan oder Titanlegierungen.

Zusätzlich können die Anlageelemente beschichtet sein, insbesondere mit einer Beschichtung, die das Anhaften von Knochenmaterial weitgehend verhindert, beispielsweise kann diese Beschichtung aus Polytetrafluorethylen bestehen.

Beim Anlegen des Sternumverschlusses 1 der Figuren 1 bis 3 werden zunächst bei auseinandergespreizten Sternumteilen 11, 12 die beiden Teile 2, 3 des inneren Anlageelementes 4 mit den in diese eingesetzten Raststiften 6 an der Innenfläche der beiden Sternumteile 11, 12 so positioniert, daß die Raststifte 6 längs des Randes der Sternumteile 11, 12 angeordnet sind, und durch ein Andrücken der Teile 2, 3 an die Innenfläche der Sternumteile 11, 12 werden die Vorsprünge 10 der Teile 2, 3 in das Knochenmaterial eingegraben. Dadurch werden die Teile 2, 3 an der Innenfläche der Sternumteile 11, 12 anliegend an diesen gehalten.

Die Positionierung der beiden Teile 2, 3 an den Sternumteilen 11, 12 erfolgt dabei so, daß die Verbindungsbereiche 8 der beiden Teile 2, 3 in Längsrichtung des Zwischenraums 22 zwischen den beiden Sternumteilen 11, 12 gegeneinander versetzt sind, d.h. die Verbindungsbereiche 8 des einen Teiles 2 greifen zwischen die Verbindungsbereiche 8 des anderen Teiles 3 ein. Diese Positionierung kann beispielsweise mit Hilfe einer in der Zeichnung nicht dargestellten Schablone erreicht werden.

In einem nächsten Schritt werden die beiden Sternumteile 11, 12 mit Hilfe einer in der Zeichnung nicht dargestellten Spanneinrichtung gegeneinander gespannt, beispielsweise mit Hilfe einer Spannzange, so daß die Breite des Zwischenraumes 22 verringert wird. Die beiden Teile 2, 3 werden dadurch ebenfalls einander angenähert, bis die Raststifte 6 der beiden Teile 2, 3 annähernd in einer Linie liegen, d.h. bis die Breite des Zwischenraumes 2 so weit reduziert ist, daß sie etwa dem Durchmesser der Raststifte 6 entspricht. In dieser Stellung wird auf jeweils zwei in Längsrichtung des Zwischenraumes 22 nebeneinander liegende Raststifte 6 jeweils ein äußeres Anlageelement 5 aufgeschoben, so daß die Verlängerungsabschnitte 16 in die Öffnungen 18, 19 eintreten. Dabei wird jedes äußere Anlageelement 5 von einem Raststift 6 durchsetzt, der an einem Teil 2 des inneren Anlageelementes 4 gehalten ist, und von einem zweiten Raststift 6, der am anderen Teil 3 des inneren Anlageelementes 4 gehalten ist.

Mittels eines ebenfalls in der Zeichnung nicht dargestellten geeigneten Werkzeuges wird das äußere Anlageelement 5 dann in Richtung auf das innere Anlageelement 4 verschoben, wobei die Lappen 21 der Öffnung 18 an den Umfangsrippen 14 des Rastabschnittes 15 der Raststifte 6 vorbeigleiten. Diese Bewegung dauert an, bis die Vorsprünge 17 des äußeren Anlageelementes 5 in das Knochenmaterial der beiden Sternumteile 11, 12 eindringen und bis das äußere Anlageelement 5 flächig an der Außenseite der beiden Sternumteile 11, 12 anliegt. In dieser Position sind inneres Anlageelement und die äußeren Anlageelemente 5 gegeneinander gespannt.

Dieser Spannvorgang kann dadurch gefördert werden, daß auf die Verlängerungsabschnitte 16 brückenförmige Anschläge 23 aufgesetzt werden, an denen das Instrument angelegt wird, welches das äußere Anlageelement 5 in Richtung auf das innere Anlageelement 4 verschiebt.

Sobald die äußeren Anlageelemente 5 gegen das innere Anlageelement 4 gespannt sind, werden die Raststifte 6 unmittelbar oberhalb der äußeren Anlageelemente 5 entfernt, beispielsweise mit Hilfe einer Schneidzange. Die äußeren Anlageelemente 5 werden durch den Eingriff der Lappen 21 in die Umfangsrippen 14 in der einmal erreichten Position festgelegt, so daß dadurch auch die beiden Sternumteile 11, 12 dauerhaft gegeneinander gespannt sind.

Bei einer Reoperation werden zunächst die äußeren Anlageelemente 5 von der Außenseite des Sternums entfernt. Dadurch werden auch die Teile 2, 3 des inneren Anlageelementes 4 voneinander getrennt, die über die als Brücke wirkenden äußeren Anlageelemente 5 über die Raststifte 6 miteinander verbunden waren.

Nach Durchtrennung des Sternums mit Hilfe einer Knochensäge können daher die Teile 11, 12 des Sternums ohne weiteres auseinandergespreizt werden, diese Bewegung wird durch das innere Anlageelement 4 in keiner Weise behindert, da die zwei Teile des inneren Anlageelementes 4 keine Verbindung mehr zueinander haben.

Nach dem Auseinanderspreizen der Teile des Sternums können die inneren Anlageelemente 4 von der Innenfläche der Sternumteile 11, 12 abgelöst und vollständig entfernt werden, so daß durch den Zwischenraum 22 ohne Behinderung durch Teile des Sternumverschlusses ein Zugang in das Körperinnere zur Verfügung steht.

Bei dem Ausführungsbeispiel der Figuren 1 bis 3 werden die Teile 2, 3 des inneren Anlageelementes 4 nur über die Raststifte 6 und die aufgesetzten äusseren Anlageelemente 5 miteinander verbunden.

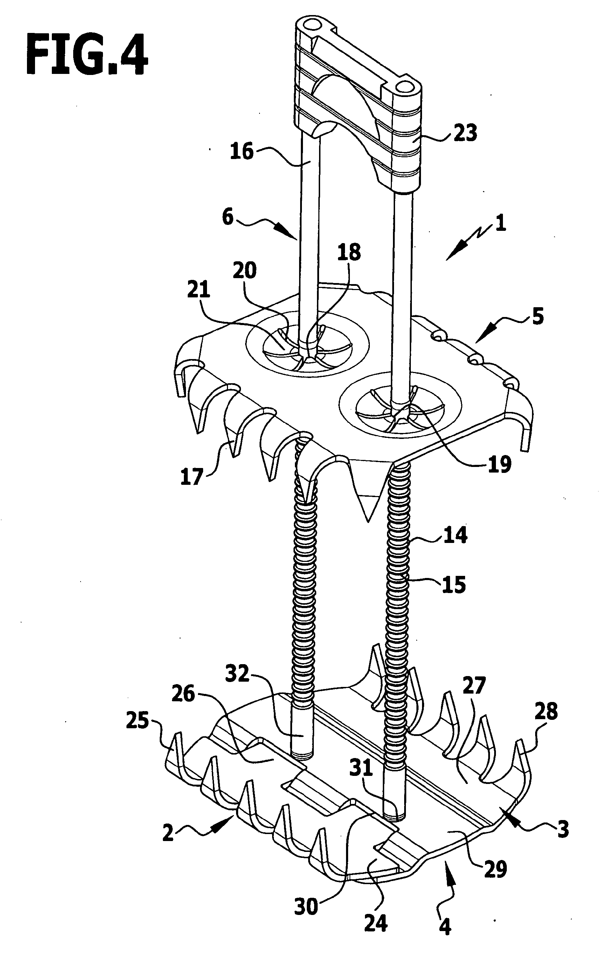

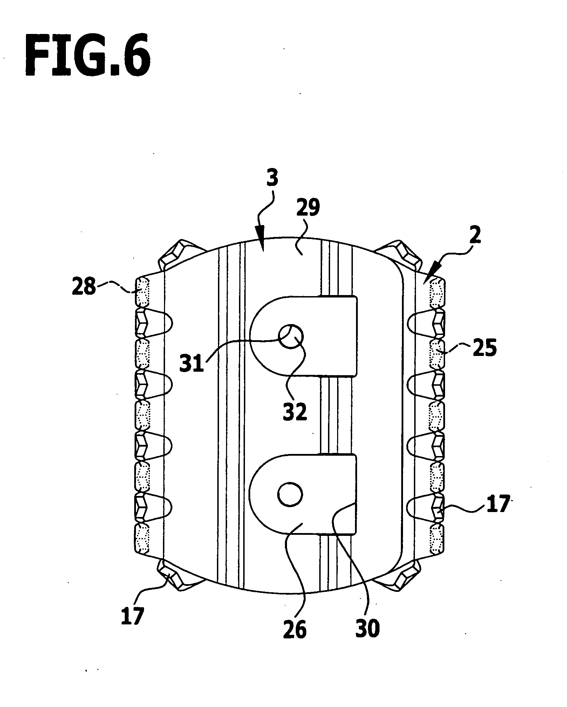

Im Gegensatz dazu weist der Sternumverschluß der Figuren 4 bis 6, bei dem entsprechende Teile dieselben Bezugszeichen tragen, ein inneres Anlageelement 4 mit zwei Teilen 2, 3 auf, die unmittelbar aneinander anliegen und lösbar miteinander verbunden sind.

Ein Teil 2 des inneren Anlageelementes 4 bildet eine ebene, im wesentlichen rechteckige Anlagefläche 24 aus, die an der Außenkante nach oben abstehende, spitze Vorsprünge 25 trägt und an der gegenüberliegenden Seite in der Ebene der Anlagefläche 24 liegende, zinkenförmige Lappen 26, die im Abstand zueinander angeordnet sind.

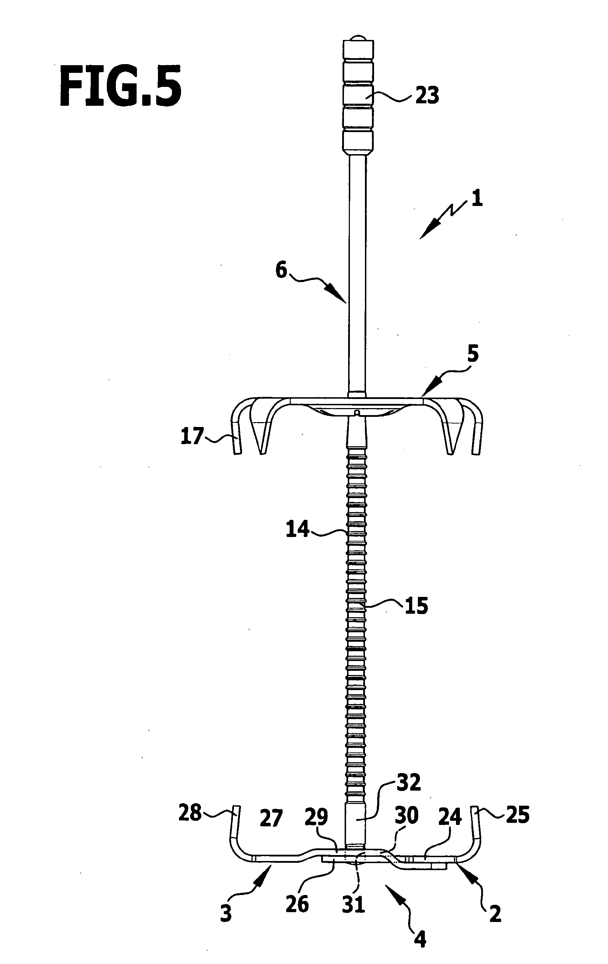

Das andere Teil 3 weist ebenfalls eine weitgehend ebene Anlagefläche 27 und an der außenliegenden Längskante nach oben abstehende, spitze Vorsprünge 28 auf, die Anlagefläche 26 ist dabei zweifache gekröpft, so daß ein geringfügig erhöhter Mittelbereich 29 entsteht (Figur 5). Im Übergangsbereich zwischen diesem Mittelbereich 29 einerseits und den Vorsprüngen 27 andererseits sind Durchstecköffnungen 30 für die Lappen 26 des Teils 2 angeordnet. Durch diese Durchstecköffnungen 30 können die Lappen 26 hindurchgesteckt werden und legen sich dann flächig an die Unterseite des erhöhten Mittelbereiches 29 an, während der den Vorsprüngen 28 gegenüberliegende Teil der Anlagefläche 27 flächig an der Unterseite des Teiles 2 zur Anlage gelangt.

Im Mittelbereich 29 und in den Lappen 26 sind Gewindebohrungen 31 angeordnet, in die das untere, ein entsprechendes Außengewinde tragende Ende 32 eines Raststiftes 6 eingeschraubt ist, so daß dadurch die beiden Teile 2,3 fest miteinander verbunden sind.

In dem dargestellten Ausführungsbeispiel weist das Teil 2 zwei nebeneinanderliegende Lappen 26 und das Teil 3 zwei entsprechende Durchstecköffnungen 30 auf, so daß zwei Raststifte 6 in das innere Anlageelement 4 eingeschraubt sind und dadurch die beiden Teile 2, 3 fest miteinander verbinden.

Der Sternumverschluß der Figuren 4 bis 6 kann in dieser Weise vormontiert werden, dabei wird das äußere Anlageelement 5 auf die Verlängerungsabschnitte 16 aufgeschoben, und die Verlängerungsabschnitte 16 werden an ihren freien Enden über einen brückenförmigen Anschlag 23 miteinander verbunden. In dieser Form kann der vormontierte Sternumverschluß 1 bei gespreizten Sternumteilen 11, 12 eingesetzt und dann in der herkömmlichen Weise nach dem Zusammenspannen der Sternumteile 11, 12 an diesen dadurch festgelegt werden, daß das innere Anlageelement 4 gegen die Innenfläche gedrückt wird. Mittels eines am Anschlag 23 angreifenden Spanninstrumentes wird dann in der beschriebenen Weise das äußere Anlageelement 5 gegen das innere Anlageelement 4 gespannt und die überstehenden Raststifte 6 entfernt.

Bei einer Reoperation werden in gleicher Weise wie bei dem Ausführungsbeispiel der Figuren 1 bis 3 zunächst die äußeren Anlageelemente 5 entfernt und anschließend mittels eines geeigneten Instrumentes die Raststifte 6 aus den Gewindebohrungen 31 herausgeschraubt. Dadurch werden die beiden Teile 2, 3 des inneren Anlageelementes 4 voneinander getrennt, so daß sie quer zur Längsrichtung des Zwischenraumes auseinandergezogen werden können, d.h. in Aufspreizrichtung der Sternumteile 11, 12. Diese Aufspreizung erfolgt nach einer Durchtrennung des Sternums mit einer Knochensäge, wobei dieser Vorgang nach der Entfernung der äußeren Anlageelemente 5 und der Raststifte 6 von den verbleibenden inneren Anlageelementen 4 in keiner Weise behindert wird.

Nach dem Aufspreizen der Sternumteile 11, 12 können die Teile 2, 3 von der Innenfläche des Sternums abgelöst und entfernt werden.

## Patentansprüche

1. Sternumverschluß zur Festlegung von zwei miteinander zu verbindenden Sternumteilen mit einem inneren Anlageelement (4) zur Anlage an der Innenfläche des Sternums, mindestens einem daran festgelegten, quer von diesem abstehenden Spannelement (6) und mit mindestens einem äußeren Anlageelement (5) zur Anlage an der Außenseite des Sternums, welches mittels des durch den Zwischenraum zwischen den Sternumteilen hindurchgeführten Spannelements (6) gegen das innere Anlageelement (4) spannbar ist, **dadurch gekennzeichnet, daß** das innere Anlageelement (4) aus zwei voneinander getrennten Teilen (2, 3) besteht, von denen jedes zur Anlage an einem der beiden Sternumteile (11, 12) ausgebildet ist, und daß Verbindungsmittel (6; 5, 6) zur lösbaren Verbindung der beiden nebeneinander angeordneten Teile (2, 3) vorgesehen sind.

2. Sternumverschluß nach Anspruch 1, **dadurch gekennzeichnet, daß** jedes Teil (2, 3) bei der Anlage an der Innenfläche der Sternumteile (11, 12) in diese eindringende Fixiervorsprünge (10; 25, 28) trägt.

3. Sternumverschluß nach einem der voranstehenden Ansprüche, **dadurch gekennzeichnet, daß** die Teile (2, 3) eine im wesentlichen ebene Anlagefläche (7; 24, 27) zur Anlage an der Innenfläche der Sternumteile (11, 12) aufweisen.

4. Sternumverschluß nach einem der voranstehenden Ansprüche, **dadurch gekennzeichnet, daß** die Spannelemente (6) stiftförmig ausgebildet sind.

5. Sternumverschluß nach einem der voranstehenden Ansprüche, **dadurch gekennzeichnet, daß** an jedem der beiden Teile (2, 3) des inneren Anlageelementes (4) Spannelemente (6) angeordnet sind und daß die äußeren Anlageelemente (5) jeweils mit Spannelementen (6) beider Teile (2, 3) des inneren Anlageelementes (4) in Verbindung stehen, so daß die beiden Teile (2, 3) des inneren Anlageelements (4) durch das äußere Anlageelement (5) nebeneinanderliegend zusammengehalten werden.

6. Sternumverschluß nach Anspruch 5, **dadurch gekennzeichnet, daß** die Spannelemente (6) in einem Bereich der Teile (2, 3) des inneren Anlageelements (4) mit diesen verbunden sind, der in Richtung auf das jeweils andere Teil (3, 2) vorspringt, und daß diese vorspringenden Bereiche (8) der Teile (2, 3) des inneren Anlageelementes (4) in Längsrichtung des Zwischenraums (22) zwischen den Sternumteilen (11, 12) gegeneinander versetzt sind.

7. Sternumverschluß nach Anspruch 6, **dadurch gekennzeichnet, daß** die vorspringenden Bereiche (8) dreieckförmig ausgebildet sind und im Bereich der Spitze des Dreiecks mit dem Spannelement (6) verbunden sind.

8. Sternumverschluß nach einem der Ansprüche 5 bis 7, **dadurch gekennzeichnet, daß** an jedem der beiden Teile (2, 3) des inneren Anlageelementes (4) in Längsrichtung des Zwischenraums (22) zwischen den Sternumteilen (11, 12) aufeinanderfolgend mehrere Spannelemente (6) angeordnet sind.

9. Sternumverschluß nach Anspruch 8, **dadurch gekennzeichnet, daß** mehrere äußere Anlageelemente (5) vorgesehen sind, von denen jedes mit je einem Spannelement (6) des einen und des anderen Teils (2, 3) des inneren Anlageelementes (4) verbunden ist.

10. Sternumverschluß nach einem der Ansprüche 1 bis 4, **dadurch gekennzeichnet, daß** die beiden Teile (2, 3) des inneren Anlageelementes (4) flächig aneinanderliegende Bereiche (29, 26) aufweisen, und daß die Verbindungsmittel (6) die beiden Teile (2, 3) in diesen Bereichen (29, 26) miteinander lösbar verbinden.

11. Sternumverschluß nach Anspruch 10, **dadurch gekennzeichnet, daß** die beiden Teile (2, 3) durch Gewindeschrauben (6) miteinander verschraubt sind.

12. Sternumverschluß nach Anspruch 11, **dadurch gekennzeichnet, daß** die Gewindeschrauben durch die Spannelemente (6) gebildet werden.

13. Sternumverschluß nach einem der Ansprüche 10 bis 12, **dadurch gekennzeichnet, daß** die beiden Teile (2, 3) des inneren Anlageelementes (4) auf den dem jeweils anderen Teil (3, 2) zugewandten Seiten zinkenförmige Vorsprünge tragen und daß diese zinkenförmigen Vorsprünge in Längsrichtung des Zwischenraumes (22) zwischen den Sternumteilen (11, 12) gegeneinander versetzt derart ineinander greifen, daß die zinkenförmigen Vorsprünge des einen Teils (2, 3) jeweils das andere Teil (3, 2) untergreifen.

14. Sternumverschluß nach einem der Ansprüche 10 bis 12, **dadurch gekennzeichnet, daß** eines der beiden Teile (2) des inneren Anlageelements (4) auf seiner dem anderen Teil (3) zugewandten Seite zinkenförmige Vorsprünge (26) trägt, die in Durchbrechungen (30) des anderen Teils (3) eintauchen und das andere Teil (3) untergreifen.

15. Sternumverschluß nach einem der Ansprüche 10 bis 14, **dadurch gekennzeichnet, daß** das innere Anlageelement (4) mit zwei Spannelementen (6) verbunden ist, die in Längsrichtung des Zwischenraums (22) zwischen den Sternumteilen (11, 12) versetzt sind.

16. Sternumverschluß nach einem der voranstehenden Ansprüche, **dadurch gekennzeichnet, daß** die Anlageelemente (4, 5) mit einem Material beschichtet sind, das die Verbindung mit Knochenmaterial verhindert.

17. Sternumverschluß nach Anspruch 16, **dadurch gekennzeichnet, daß** die Anlageelemente (4, 5) mit Polytetrafluorethylen beschichtet sind.

## Claims

1. Sternum closure device for fixing two sternum portions to be joined to one another, comprising an inner contact element (4) for abutment on the inner surface of the sternum, at least one clamping element (6) being fixed to said contact element (4) and projecting transversely from it and at least one outer contact element (5) for abutment on the outer side of the sternum, said outer contact element being clampable against the inner contact element (4) by means of the clamping, element (6) guided through the intermediate space between the sternum portions, **characterised in that** the inner contact element (4) consists of two parts (2, 3) separate from one another, each of said parts (2, 3) being designed to abut on one of the two sternum portions (11, 12) and wherein connecting elements (6; 5, 6) are provided for the releasable connection of the two parts (2, 3) arranged next to one another.

2. Sternum closure device in accordance with claim 1, **characterised in that**, during abutment on the inner surface of the sternum portions (11, 12), each part (2, 3) has fixing projections (10; 25, 28) penetrating said portions (11, 12).

3. Sternum closure device in accordance with any one of the preceding claims, **characterised in that** the parts (2, 3) have an essentially flat contact surface (7; 24, 27) for abutment on the inner surface of the sternum portions (11, 12).

4. Sternum closure device in accordance with any one of the preceding claims, **characterised in that** the clamping elements (6) are of a pin-shaped design.

5. Sternum closure device in accordance with any one of the preceding claims, **characterised in that** clamping elements (6) are arranged on each of the two parts (2, 3) of the inner contact element (4) and **in that** the outer contact elements (5) are each connected to clamping elements (6) of both parts (2, 3) of the inner contact element (4) such that the two parts (2, 3) of the inner contact element (4) are held together by the outer contact element (5), located next to one another.

6. Sternum closure device in accordance with claim 5, **characterised in that** the clamping elements (6) are connected to the parts (2, 3) of the inner contact element (4) in an area of the parts (2, 3) projecting in the direction towards the respectively other part (3, 2) and **in that** these projecting areas (8) of the parts (2, 3) of the inner contact element (4) are offset relative to one another in longitudinal direction of the intermediate space (22) between the sternum portions (11, 12).

7. Sternum closure device in accordance with claim 6, **characterised in that** the projecting areas (8) are of a triangular design and are connected to the clamping element (6) in the area of the tip of the triangle.

8. Sternum closure device in accordance with any one of claims 5 to 7, **characterised in that** a plurality of clamping elements (6) are arranged on each of the two parts (2, 3) of the inner contact element (4) so as to follow one another in longitudinal direction of the intermediate space (22) between the sternum portions (11, 12).

9. Sternum closure device in accordance with claim 8, **characterised in that** a plurality of outer contact elements (5) are provided, each of said contact elements (5) being connected to a clamping element (6) of both the one and the other parts (2, 3) of the inner contact element (4).

10. Sternum closure device in accordance with any one of claims 1 to 4, **characterised in that** the two parts (2, 3) of the inner contact element (4) have areas (29, 26) abutting areally on one another and **in that** the connecting elements (6) releasably connect the two parts (2, 3) to one another in these areas (29, 26).

11. Sternum closure device in accordance with claim 10, **characterised in that** the two parts (2, 3) are screwed to one another by threaded screws (6).

12. Sternum closure device in accordance with claim 11, **characterised in that** the threaded screws are formed by the clamping elements (6).

13. Sternum closure device in accordance with any of claims 10 to 12, **characterised in that** the two parts (2, 3) of the inner contact element (4) have prong-like projections on the sides facing the respectively other part (3, 2) and **in that** these prong-like projections are offset relative to one another in longitudinal direction of the intermediate space (22) between the sternum portions (11, 12) and engage in one another in such a manner that the prong-like projections of the one part (2, 3) engage under the respectively other part (3, 2).

14. Sternum closure device in accordance with any of claims 10 to 12, **characterised in that** one of the two parts (2) of the inner contact element (4) has prong-like projections (26) on its side facing the other part (3), said projections (26) dipping into recesses (30) in the other part (3) and engaging beneath the other part (3).

15. Sternum closure device in accordance with any of claims 10 to 14, **characterised in that** the inner contact element (4) is connected to two clamping elements (6) offset in longitudinal direction of the intermediate space (22) between the sternum portions (11, 12).

16. Sternum closure device in accordance with any of the preceding claims, **characterised in that** the contact elements (4, 5) are coated with a material preventing the bonding to bone material.

17. Sternum closure device in accordance with claim 16, **characterised in that** the contact elements (4, 5) are coated with polytetrafluoroethylene.

## Revendications

1. Dispositif de fermeture sternale pour l'immobilisation de deux parties de sternum à relier l'une à l'autre, comprenant un élément d'appui intérieur (4) destiné à venir s'appuyer sur la surface intérieure du sternum, au moins un élément de serrage (6) qui est fixé à l'élément d'appui intérieur et en fait saillie transversalement, et comprenant également au moins un élément d'appui extérieur (5), qui est destiné à venir s'appuyer sur le côté extérieur du sternum, et peut être serré vers l'élément d'appui intérieur (4) au moyen de l'élément de serrage (6) passant à travers l'espace intermédiaire entre les parties de sternum,
**caractérisé en ce que** l'élément d'appui intérieur (4) est constitué de deux pièces (2, 3) séparées l'une de l'autre et dont chacune est conçue pour s'appuyer contre l'une des deux parties de sternum (11, 12), et **en ce que** sont prévus des moyens de liaison (6 ; 5, 6) permettant d'assurer une liaison amovible des deux pièces (2, 3) agencées l'une à côté de l'autre.

2. Dispositif de fermeture sternale selon la revendication 1, **caractérisé en ce que** chaque pièce (2, 3) porte, pour son appui sur la surface intérieure des parties de sternum (11, 12), des protubérances de fixation (10 ; 25, 28) pénétrant dans ces parties de sternum.

3. Dispositif de fermeture sternale selon l'une des revendications précédentes, **caractérisé en ce que** les pièces (2, 3) présentent une surface d'appui (7 ; 24, 27) sensiblement plane pour venir s'appuyer sur la surface intérieure des parties de sternum (11, 12).

4. Dispositif de fermeture sternale selon l'une des revendications précédentes, **caractérisé en ce que** les éléments de serrage (6) sont d'une configuration en forme de broche.

5. Dispositif de fermeture sternale selon l'une des revendications précédentes, **caractérisé en ce que** sur chacune des deux pièces (2, 3) de l'élément d'appui intérieur (4) sont agencés des éléments de serrage (6), et **en ce que** les éléments d'appui extérieurs (5) sont chacun en liaison avec des éléments de serrage (6) des deux pièces (2, 3) de l'élément d'appui intérieur (4), de sorte que les deux pièces (2, 3) de l'élément d'appui intérieur (4) sont maintenues assemblées côte à côte par l'élément d'appui extérieur (5).

6. Dispositif de fermeture sternale selon la revendication 5, **caractérisé en ce que** les éléments de serrage (6) sont liés à l'élément d'appui intérieur (4) dans une zone des pièces (2, 3) de celui-ci, qui fait saillie en direction de respectivement l'autre pièce (3, 2), et **en ce que** ces zones (8) en saillie des pièces (2, 3) de l'élément d'appui intérieur (4), sont mutuellement décalées les unes par rapport aux autres dans la direction longitudinale de l'espace intermédiaire (22) entre les parties de sternum (11, 12).

7. Dispositif de fermeture sternale selon la revendication 6, **caractérisé en ce que** les zones (8) en saillie sont d'une configuration en forme de triangle, et sont liées avec l'élément de serrage (6) dans la zone du sommet du triangle.

8. Dispositif de fermeture sternale selon l'une des revendications 5 à 7, **caractérisé en ce que** sur chacune des deux pièces (2, 3) de l'élément d'appui intérieur (4), sont agencés successivement, dans la direction longitudinale de l'espace intermédiaire (22) entre les parties de sternum (11, 12), plusieurs éléments de serrage (6).

9. Dispositif de fermeture sternale selon la revendication 8, **caractérisé en ce que** sont prévus plusieurs éléments d'appui extérieurs (5) dont chacun est relié avec un élément de serrage (6) de l'une et de l'autre des pièces (2, 3) de l'élément d'appui intérieur (4) .

10. Dispositif de fermeture sternale selon l'une des revendications 1 à 4, **caractérisé en ce que** les deux pièces (2, 3) de l'élément d'appui intérieur (4) présentent des zones (29, 26) s'appuyant l'une contre l'autre de manière surfacique, et **en ce que** les moyens de liaison (6) relient de façon amovible les deux pièces (2, 3) au niveau de ces zones (29, 26).

11. Dispositif de fermeture sternale selon la revendication 10, **caractérisé en ce que** les deux pièces (2, 3) sont vissées l'une à l'autre par des vis filetées (6).

12. Dispositif de fermeture sternale selon la revendication 11, **caractérisé en ce que** les vis filetées sont formées par les éléments de serrage (6).

13. Dispositif de fermeture sternale selon l'une des revendications 10 à 12, **caractérisé en ce que** les deux pièces (2, 3) de l'élément d'appui intérieur (4) portent des protubérances en forme de dent ou de griffe sur les côtés dirigés respectivement vers l'autre pièce (3, 2), et **en ce que** ces protubérances en forme griffe, mutuellement décalées dans la direction longitudinale de l'espace intermédiaire (22) entre les parties de sternum (11, 12), engrènent les unes avec les autres de façon telle que les protubérances en forme de griffe de l'une des pièces (2, 3) s'engagent sous respectivement l'autre pièce (3, 2).

14. Dispositif de fermeture sternale selon l'une des revendications 10 à 12, **caractérisé en ce que** l'une des deux pièces (2) de l'élément d'appui intérieur (4) porte sur son côté dirigé vers l'autre pièce (3), des protubérances (26) en forme de griffe, qui pénètrent dans des ouvertures de passage (30) de l'autre pièce (3) et s'engagent sous ladite autre pièce (3).

15. Dispositif de fermeture sternale selon l'une des revendications 10 à 14, **caractérisé en ce que** l'élément d'appui intérieur (4) est relié à deux éléments de serrage (6), qui sont mutuellement décalés l'un par rapport à l'autre dans la direction longitudinale de l'espace intermédiaire (22) entre les parties de sternum (11, 12).

16. Dispositif de fermeture sternale selon l'une des revendications précédentes, **caractérisé en ce que** les éléments d'appui (4, 5) sont revêtus d'un matériau, qui empêche une liaison avec la matière osseuse.

17. Dispositif de fermeture sternale selon la revendication 16, **caractérisé en ce que** les éléments d'appui (4, 5) sont revêtus de polytétrafluoroéthylène.
